(19)

**Europäisches Patentamt**
**European Patent Office**
**Office européen des brevets**

(11) **EP 1 749 575 A1**

(12) # EUROPEAN PATENT APPLICATION

(43) Date of publication:
**07.02.2007 Bulletin 2007/06**

(21) Application number: **06253938.2**

(22) Date of filing: **27.07.2006**

(51) Int Cl.:
*B01L 3/00* (2006.01)     *G01N 33/487* (2006.01)
*A61B 5/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.07.2005 US 193704**

(71) Applicant: **Lifescan Scotland Limited Inverness-Shire IV2 3ED (GB)**

(72) Inventors:
• **Stout, Phil**
  **Roseville, Minnesota 55113 (US)**
• **Lillie, Geoffrey**
  **Inverness, Inverness-shire IV3 5JW (GB)**
• **Moffat, James**
  **Inverness-shire IV3 8QU (GB)**

(74) Representative: **Tunstall, Christopher Stephen et al**
**Carpmaels & Ransford,**
**43-45 Bloomsbury Square**
**London WC1A 2RA (GB)**

(54) **Method for feedback control of a microfluidic system**

(57)     A method for the feedback control of a microfluidic system includes measuring an electrical characteristic (such as impedance or resistance) of a position electrode(s) of the microfluidic system with a meter of the microfluidic system. A feedback controller is then employed to control the flow of bodily fluid sample through the microfluidic system based on the electrical characteristic measured by the meter.

FIG.1

EP 1 749 575 A1

## Description

## CROSS-REFERENCE

This application is a continuation-in-part application of Application No. 10/811,446, filed March 26, 2004, which is incorporated herein by reference in its entirety and to which application we claim priority under 35 USC §120.

## BACKGROUND OF THE INVENTION

### 1. Field of the Invention

The present invention relates, in general, to analytical devices and, in particular, to microfluidic analytical systems.

### 2. Description of the Related Art

In analytical devices based on liquid samples (i.e., fluidic analytical devices), the requisite liquid samples should be controlled with a high degree of accuracy and precision in order to obtain reliable analytical results. Such control is especially warranted with respect to "microfluidic" analytical devices that employ liquid samples of small volume, for example, 10 nanoliters to 10 microliters. In such microfluidic analytical devices, the liquid samples are typically contained and transported in microchannels with dimensions on the order of, for example, 10 micrometers to 500 micrometers.

The control (e.g., transportation, position detection, flow rate determination and/or volume determination) of small volume liquid samples within micro-channels can be essential in the success of a variety of analytical procedures including the determination of glucose concentration in interstitial fluid (ISF) samples. For example, obtaining reliable results may require knowledge of liquid sample position and/or volume in order to insure that a sufficient liquid sample has arrived at a detection area before analysis is commenced. The relatively small size of the liquid samples and micro-channels in microfluidic analytical devices can, however, render such control problematic.

In the context of analytical systems for blood glucose monitoring, continuous or semi-continuous monitoring systems and methods are advantageous in that they provide enhanced insight into blood glucose concentration trends, the effect of food and medication on blood glucose concentration and a user's overall glycemic control. A challenge of continuous or semi-continuous glucose monitoring systems is that only small volumes of liquid sample (e.g., an ISF liquid sample of about 250 nanoliters) are generally available for measuring a glucose concentration. In addition, it is difficult to transport small volumes of liquid from a target site to an *ex vivo* glucose monitor with a controlled flow rate and in such a way that the position and total volume of extracted fluid is known.

## BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a simplified cross-sectional side view and schematic representation of a microfluidic analytical system according to an exemplary embodiment of the present invention;

FIG. 2 is a simplified perspective view of a molded plug of the microfluidic analytical system of FIG. 1;

FIG. 3 is a simplified top view of a micro-channel disc of the microfluidic analytical system of FIG. 1;

FIG. 4 is a simplified bottom view of a laminate layer of the microfluidic analytical system of FIG. 1;

FIG. 5 is a simplified block diagram depicting a microfluidic system for extracting a bodily fluid sample and monitoring an analyte therein, with which embodiments of microfluidic analytical systems according to the present invention can be employed;

FIG. 6 is a simplified schematic diagram of the sampling module of FIG. 5 being applied to a user's skin layer, with the dashed arrow indicating a mechanical interaction and the solid arrows indicating ISF flow or, when associated with element 228, the application of pressure;

FIG. 7 is a simplified schematic diagram of a position electrode, micro-channel, analyte sensor and meter configuration for use in embodiments of microfluidic analytical systems according to the present invention;

FIG. 8A is a simplified cross-sectional and schematic diagram illustrating a manner in which a position electrode can be exposed to a micro-channel in embodiments of microfluidic analytical systems according to the present invention;

FIG. 8B is a simplified cross-sectional and schematic diagram illustrating a manner in which a position electrode is separated from a micro-channel by an insulating layer in embodiments of microfluidic analytical systems according to the present invention;

FIG. 9 is simplified schematic diagram of another position electrode, microchannel, analyte sensor and meter configuration for use in embodiments of microfluidic analytical systems according to the present invention illustrating a manner by which a position detector can be in electrical communication with an analyte sensor;

FIG. 10 simplified schematic diagram of yet another position electrode, microchannel, analyte sensor and meter configuration for use in embodiments of microfluidic analytical systems according to the present invention showing the use of three position electrodes;

FIG. 11 is simplified schematic diagram of a position electrode, main microchannel, branch micro-channels, analyte sensor and meter configuration for use in embodiments of microfluidic analytical systems according to the present invention;

FIG. 12 is simplified schematic diagram of another position electrode, main micro-channel, branch micro-channels, analyte sensor and meter configuration for use in embodiments of microfluidic analytical systems according to the present invention;

FIG. 13 is a simplified schematic diagram of a position electrode, microchannel and meter configuration for use in embodiments of microfluidic analytical systems according to the present invention;

FIG. 14 is a simplified schematic diagram of an equivalent electrical circuit for a portion of the configuration of FIG. 13;

FIG. 15 is a simplified schematic diagram of a further position electrode, microchannel and meter configuration for use in embodiments of microfluidic analytical systems according to the present invention;

FIG. 16 is a simplified schematic diagram of an equivalent electrical circuit for a portion of the configuration of FIG. 15;

FIG. 17 is a simplified schematic diagram of another position electrode, microchannel and meter configuration for use in embodiments of microfluidic analytical systems according to the present invention;

FIG. 18 is a simplified schematic diagram of yet another position electrode, micro-channel and meter configuration for use in embodiments of microfluidic analytical systems according to the present invention;

FIG. 19 is a simplified schematic diagram of an equivalent electrical circuit for a portion of the configuration of FIG. 18;

FIG. 20 is a simplified schematic diagram of still another a position electrode, micro-channel and meter configuration for use in embodiments of microfluidic analytical systems according to the present invention;

FIG. 21 is a graph of admittance versus bolus number; and

FIG. 22 is a flow diagram depicting stages in a process for the feedback control of a microfluidic system according to an exemplary embodiment of the present invention.

## DETAILED DESCRIPTION OF PREFERRED EMBODIMENTS

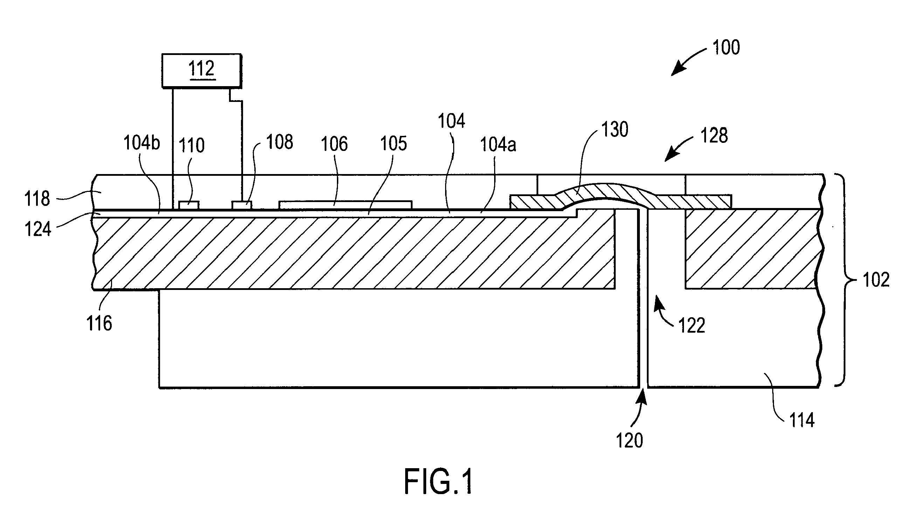

FIGs. 1-4 depict a microfluidic analytical system 100 for determining an analyte (e.g., detecting the analyte and/or measuring the concentration of the analyte) in a liquid sample according to an exemplary embodiment of the present invention.

Microfluidic analytical system 100 includes an analysis module 102 with a micro-channel 104 for receiving and transporting a liquid sample (e.g., an ISF sample extracted from a dermal tissue target site of a user's body), an analyte sensor 106 (e.g., an electrochemical analyte sensor or photometric analyte sensor) for measuring an analyte (e.g., glucose) in the liquid sample, and first and second position electrodes 108 and 110. In the embodiment of FIGs. 1-4, micro-channel 104 includes a pre-sensor micro-channel portion 104a and a post-sensor micro-channel portion 104b. Microfluidic analytical system 100 also includes a sensor chamber 105, within which analyte sensor 106 is disposed.

Microfluidic analytical system 100 further includes a meter 112 for measuring impedance between first position electrode 108 and second position electrode 110, with the measured impedance being dependent on the position of a liquid sample (not shown in FIGs. 1-4) in micro-channel 104.

In general, measuring impedances, or ohmic resistances, between position electrodes in embodiments of the present invention can be accomplished by applying a voltage therebetween and measuring the resulting current. Either a constant voltage or an alternating voltage can be applied between the position electrodes and the resulting direct current (DC) or alternating current (AC), respectively, measured. The resulting DC or AC current can then be used to calculate the impedance or ohmic resistance. Furthermore, one skilled in the art will recognize that measuring an impedance can involve measuring both an ohmic drop (i.e., resistance [R] in Ohms or voltage/current) and measuring capacitance (i.e., capacitance in Farads or coulombs/volt). In practice, impedance can be measured, for example, by applying an alternating current to the position electrode(s) and measuring the resulting current. At different frequencies of alternating current, either resistive or capacitive effects prevail in determining the measured impedance. The pure resistive component can prevail at lower frequencies while the pure capacitive component can prevail at higher frequencies. To distinguish between the resistive and capacitive components, the phase difference between the applied alternating current and the measured resulting current can be determined. If there is zero phase shift, the pure resistive component is prevailing. If the phase shift indicates that the current lags the voltage, then the capacitive component is significant. Therefore, depending on the frequency of an applied alternating current and position electrode configuration, it can be beneficial to measure either resistance or a combination of resistance and capacitance.

In the embodiment of FIGs. 1-4, impedance measurements can be performed by, for example, applying an alternating voltage between first position electrode 108 and second position electrode 110 and measuring the resulting alternating current. Since first position electrode 108 and second position electrode 110 are a portion of a capacitor (along with any substance [e.g., air or a liquid sample] within micro-channel 104 between the first and second position electrodes and any layers that may be separating the position electrodes from direct contact with the substance), the measured current can be used to calculate the impedance. The presence or absence of a liquid sample in micro-channel 104 between the first and second position electrodes will affect the measured current and impedance.

The frequency and amplitude of the alternating voltage applied between the first and second position electrodes can be predetermined such that the presence of a liquid sample between the first and second position electrodes can be detected by a significant increase in measured current.

With respect to the measurement of impedance or resistance, the magnitude of the applied voltage can be, for example, in range from about 10 mV to about 2 volts for the circumstance of an ISF liquid sample and carbon-based or silver-based ink position electrodes. The lower and upper limits of the applied voltage range are dependent on the onset of electrolysis or electrochemical decomposition of the liquid sample. In the circumstance that an alternating voltage is employed, the alternating voltage can be applied, for example, at a frequency that results in a negligible net change in the liquid sample's properties due to any electrochemical reaction. Such a frequency range can be, for example, from about 10 Hz to about 100 kHz with a voltage waveform symmetrical around 0 Volts (i.e., the RMS value of the alternating voltage is approximately zero).

As depicted in a simplified manner in FIG. 1, analyte sensor 106, first position electrode 108 and second position electrode 110 are each in operative communication with micro-channel 104. It should be noted that position electrodes employed in embodiments of the present invention can be formed of any suitable conductive material known to those skilled in the art, including conductive materials conventionally used as analytical electrode materials and, in particular, conductive materials known as suitable for use in flexible circuits, photolithographic manufacturing techniques, screen printing techniques and flexo-printing techniques. Suitable conductive materials include, for example, carbon, noble metals (e.g., gold, platinum and palladium), noble metal alloys, conductive potential-forming metal oxides and metal salts. Position electrodes can be formed, for example, from conductive silver ink, such as the commercially available conductive silver ink Electrodag 418 SS.

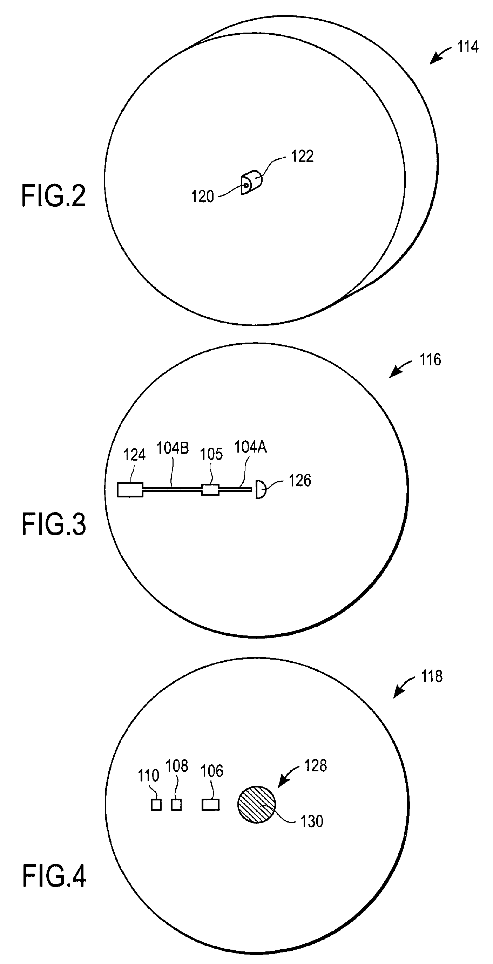

In the embodiment of FIGs. 1-4, analysis module 102 further includes a molded plug 114, a micro-channel disc 116 and a laminate layer 118 (depicted individually in FIGs. 2, 3 and 4, respectively). Analysis module 102 can be constructed by, for example, interfacing micro-channel disc 116 with laminate layer 118 and molded plug 114.

Molded plug 114 includes an inlet channel 120 and a registration pole 122. Micro-channel disc 116 is configured to define (along with laminate layer 118) a liquid sample waste reservoir 124, as well as the aforementioned micro-channel 104 and sensor chamber 105. In addition, micro-channel disc 116 includes a registration hole 126 (see, for example, FIG. 3).

Laminate layer 118 includes an access hole 128, a membrane valve 130, and in the embodiment of FIGs. 1-4, the aforementioned analyte sensor 106 and first and second position electrodes 108 and 110.

Micro-channel 104 has cross-sectional dimensions perpendicular to a direction of fluid flow (i.e., height and width) in the range of about 10 micrometers to about 500 micrometers. Typical liquid sample volumes to be handled in a micro-channel(s) of embodiments of the present invention are on the order of about 10 nanoliters to about 10 microliters. In this respect, the term "handled" is in reference to the transportation and control of various liquid sample volumes including, but not limited to, isolated liquid sample volumes extracted from a target site (e.g., isolated volumes in the range of 50 nl to 250 nl), the minimum liquid sample volume required by an analyte sensor (for example, 50 nl), and the total liquid sample volume that is conducted through a micro-channel throughout the useful lifetime of a microfluidic analytical system (for example, a total volume of approximately 10 micro-liters).

Registration pole 122 of molded plug 114 is employed during manufacturing of microfluidic analytical system 100 to ensure adequate alignment (i.e., registration) of molded plug 114 and micro-channel disc 116. For example, such alignment must insure that analyte sensor 106 is operatively aligned with sensor chamber 105 and that first and second position electrodes 108 and 110 are aligned with post-sensor microchannel channel 104b. During manufacturing, laminate layer 118 can be aligned with micro-channel disc 116 using registration features included in laminate layer 118 and/or micro-channel disc 116 (not shown) or by optical verification.

Registration hole 126 of micro-channel disc 116 is depicted as having a half circle shape and extending entirely through micro-channel disc 116. Registration pole 122 has a shape and size that are complementary to registration hole 126, thus providing for micro-channel disc 116 to securely interface with the molded plug 114, as depicted in FIG. 1. The use of half circle shapes for both registration hole 126 and registration pole 122 beneficially limits the rotational freedom of a combined molded plug 114 and micro-channel disc 116. It should be noted that alternative shapes other than a half circle can also be used.

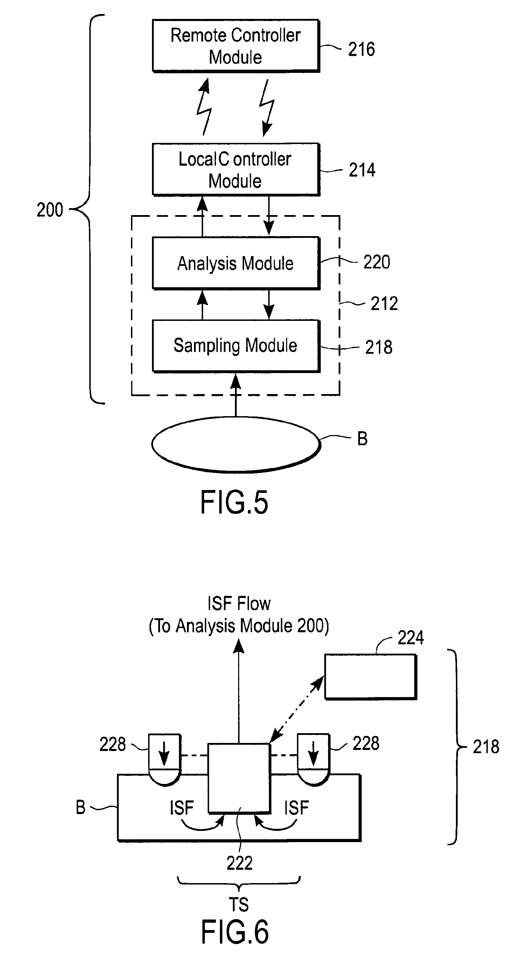

Although not depicted in FIGs. 1-4, laminate layer 118 includes electrical connections to electrically connect analyte sensor 106 to external apparatus (such as a local controller module as described with respect to FIG. 5 below) and to connect first and second position electrodes 108 and 110 to meter 112. Such electrical connections can include, for example, conductive traces and electrical contact pads.

It is contemplated that a liquid sample (e.g., an ISF sample) will be transported to inlet channel 120 by suitable means, such as a sampling module as described below with respect to FIG. 5. Flow of liquid sample through inlet channel 120 is controlled by membrane valve 130. It should be noted that other types of valves, besides membrane valves, can be used and are well know to one skilled in the art.

In an embodiment of FIG. 1, membrane valve 130 is deformable and made of an elastomeric material in a dome shape. When membrane valve 130 is an undeformed condition, a liquid sample may flow past membrane valve 130 and fill pre-sensor micro-channel portion 104a. However, when membrane valve 130 is initially deformed (for example, by the application of pressure via access hole 128), it occludes inlet channel 120 and prevents liquid sample from flowing therethrough. In addition, further deformation of membrane valve 130 pushes liquid sample through pre-sensor microchannel portion 104a and into sensor chamber 105. The movement of liquid sample past membrane valve 130 (i.e., from inlet channel 120 to pre-sensor micro-channel portion 104a) can be controlled by the amount of pressure applied in deforming membrane valve 130. Typical liquid sample flow rates into micro-channel 104 are in the range of about 10 nanoliters per minute to about 1000 nanoliters per minute.

First and second position electrodes 108 and 110, along with meter 112, can be used to determine the liquid sample position within micro-channel 104, the flow rate of a liquid sample and/or the volume of an extracted liquid sample to help control the depression of membrane valve 130. For example, upon determination of the liquid sample position, knowledge of elapsed time and micro-channel volume can be employed to calculate the flow rate and/or volume of the liquid sample. One skilled in the art will recognize, therefore, that, in general, position electrode(s) electrical characteristics (such as impedance and resistance) measured by meter 112 are indicative of liquid sample position, liquid sample flow rate and liquid sample volume.

In addition, meter 112 can optionally include an integrated feedback controller (e.g., a feedback controller that includes suitable microprocessors and/or other electronic circuits) configured to provide a feedback control loop that places membrane valve 130 in a deformed or undeformed condition depending on the flow rate, position or volume of liquid sample within microchannel 104. In other words, such a feedback control loop serves to occlude (close) or open inlet channel 120 to the flow of liquid sample therethrough. Once apprised of the present disclosure, one skilled in the art can readily ascertain suitable feedback controllers for providing such a feedback control loop.

The use of a feedback controller has the benefit of enabling the collection of the minimal volume of liquid sample required to perform an accurate measurement of the analyte and in doing so, to minimize measurement time across varying sampling conditions.

It can be beneficial to determine liquid sample position in order to, for example, ascertain when a minimum amount of liquid sample has entered analysis module 102 in order to initiate analyte determination. It can also be beneficial to determine liquid sample flow rate and/or the total amount of liquid sample that has entered microfluidic analytical system 100 in order to control membrane valve 130 in a manner that facilitates semi-continuous stopped flow measurements (i.e., measurements taken with liquid sample flow momentarily halted and that result in a predetermined number of measurements per unit time [typically in the range of 4 to 10 measurements per hour] rather than a continuous measurement) over predetermined time periods. In addition, determining liquid sample flow rate and the total amount of liquid sample enables sensor lag compensation. Furthermore, analyte sensor 106 may be sensitive to flow rate. Therefore, the use of first and second position electrodes, along with meter 112, allows system 100 to more accurately determine an analyte over an extended period of time such as, for example, about 8 hours. As described above, membrane valve 130 can be controlled by a feedback controller to facilitate the aforementioned semi-continuous stopped flow measurements.

In the embodiment of FIGs. 1-4, analyte sensor 106 is disposed within sensor chamber 105. Analyte sensor 106 can be any suitable sensor known to one skilled in the art. For the circumstance where the analyte of interest is glucose, analyte sensor 106 can be an electrochemical glucose sensor that measures a current proportional to glucose concentration. More particularly, analyte sensor 106 can be, for example, an electrochemical glucose sensor that measures current under stopped flow conditions (i.e., flow rates at or near zero during measurement) and with glucose being consumed within sensor chamber 105. Examples of analyte sensors that may be used in embodiments of the present invention include, but not limited to, electrochemical-based and photometric-based analyte sensors. Electrochemical-based analyte sensors include, for example, amperometric, potentiometric and coulometric analyte sensors. Photometric-based analyte sensors include, for example, transmission, reflectance, colorimetric, fluorometric, scattering and absorbance analyte sensors.

After an analyte in a liquid sample has been determined by analyte sensor 106, the liquid sample is transported to post-sensor micro-channel portion 104b.

One skilled in the art will recognize that analyte monitoring systems according to embodiments of the present invention can be employed, for example, as a subsystem in a variety of devices. For example, embodiments of the present invention can be employed as an analysis module of microfluidic system 200 depicted in FIG. 5. Microfluidic system 200 is configured for extracting a bodily fluid sample (e.g., an ISF sample) and monitoring an analyte (e.g., glucose) therein. System 200 includes a disposable cartridge 212 (encompassed within the dashed box), a local controller module 214 and a remote controller module 216. It should be noted that in FIG. 5, the arrows indicate direction of transfer for both liquid sample and electrical signals as the appropriate circumstances dictate.

In system 200, disposable cartridge 212 includes a sampling module 218 for extracting the bodily fluid sample (namely, an ISF sample) from a body (B, for example, a user's skin layer) and an analysis module 200 for measuring an analyte (i.e., glucose) in the bodily fluid. Sampling module 218 can be any suitable sampling module known to those of skill in the art, while analysis module 220 can be a microfluidic analytical system according to embodiments of the present invention. Examples of suitable sampling modules are described in International Application PCT/GB01/05634 (published as WO 02/49507 A1 on June 27, 2002) and U.S. Patent Application No. 10/653,023, which is hereby fully incorporated herein by reference. However, in system 200, sampling module 218 is configured to be disposable since it is a component of disposable cartridge 212.

As depicted in FIG. 6, the sampling module 218 of system 200 is an ISF sampling module that includes a penetration member 222 for penetrating a target site (TS) of body B and extracting an ISF sample, a launching mechanism 224 and at least one pressure ring 228. Sampling module 218 is adapted to provide a continuous or semi-continuous flow of ISF to analysis module 220 for the monitoring (e.g., concentration measurement) of an analyte (such as glucose) in the ISF sample.

During use of system 200, penetration member 222 is inserted into the target site (i.e., penetrates the target site) by operation of launching mechanism 224. For the extraction of an ISF sample from a user's skin layer, penetration member 222 can be inserted to a maximum insertion depth in the range of, for example, 1.5 mm to 3 mm. In addition, penetration member 222 can be configured to optimize extraction of an ISF sample in a continuous or semi-continuous manner. In this regard, penetration member 222 can include, for example, a 25 gauge, thin-wall stainless steel needle (not shown in FIGs. 5 or 6) with a bent tip, wherein a fulcrum for the tip bend is disposed between the needle's tip and the needle's heel. Suitable needles for use in penetration members are described in U.S. Patent Application No. 10/185,605 (published as US 2003/0060784 A1 on March 27, 2003). Furthermore, further details regarding system 200 are in U.S. Patent Application No. 10/718,818.

Once penetration member 222 has penetrated target site TS, pressure ring(s) 228 can be employed to apply pressure (i.e., apply force) to the user's skin layer in the vicinity of the target site (indicated by the downward pointing arrows of FIG. 6). Such pressure serves to create a sub-dermal interstitial fluid (ISF) pressure gradient in the vicinity of the target site. This sub-dermal ISF pressure gradient induces a flow of ISF through penetration member 222 and sampling module 218 to analysis module 220 (as indicated by the curved and upward pointing arrows of FIG. 6).

Pressure ring 228 can be referred to as being in a "deployed" state when pressure ring 228 is applying pressure on the user's skin layer in the vicinity of the target site as described above. Furthermore, pressure ring 228 can be referred to as being in a "retracted" state when pressure ring 228 is not applying pressure to the user's skin layer in the vicinity of the target site, although the penetration member can continue to reside in the user's skin layer. In such a retracted state, fluid flow through penetration member 222 is effectively stopped.

Therefore, placing pressure ring 228 in a deployed or retracted state can serve to control (e.g., start, stop or otherwise adjust) the flow of liquid sample (e.g., ISF sample) through sampling module 218 and into analysis module 220. Placing pressure ring 228 into a deployed and/or retracted state can be accomplished via a feedback controller adapted to control the flow of bodily fluid sample through the microfluidic system based on an electrical characteristic (such as impedance or resistance) measured by the meter. Such a feedback controller can, therefore, be employed to, for example, facilitate semi-continuous stopped flow measurements.

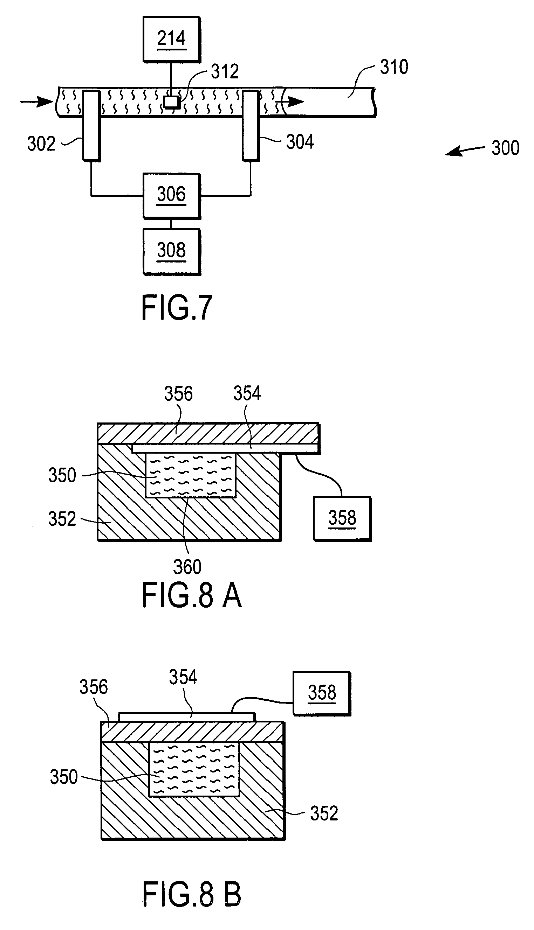

In the embodiment of FIGs. 1-4, first and second position electrodes 108 and 110 and meter 112 are configured such that both the first and second position electrodes are "downstream" of analyte sensor 106 with respect to micro-channel 104. However, other suitable configurations can be employed. For example, FIG. 7 is a simplified schematic diagram of a position electrode, micro-channel, analyte sensor and meter configuration 300 for use in embodiments of microfluidic analytical systems according to the present invention. Configuration 300 includes first position electrode 302, second position electrode 304, electrical impedance meter 306, timer 308, microchannel 310 and analyte sensor 312. In the configuration of FIG. 7, wavy lines depict a liquid sample (e.g., an ISF, blood, urine, plasma, serum, buffer or reagent liquid sample) within micro-channel 310.

Configuration 300 can be used to determine the position or flow rate of a liquid sample in micro-channel 310. In the configuration of FIG. 7, analyte sensor 312 is located in-between first position electrode 302 and second position electrode 304. Electrical impedance meter 306 is adapted for measuring an electrical impedance between first position electrode 302 and second electrode 304. Such a measurement can be accomplished by, for example, employing a voltage source to impose either a continuous or alternating voltage between first position electrode 302 and second position electrode 304 such that an impedance resulting from a conducting path formed by a liquid sample within micro-channel 310 and between first position electrode 302 and second position electrode 304 can be measured, yielding a signal indicative of the presence of the liquid sample.

Furthermore, when electrical impedance meter 306 measures a change in impedance due to the presence of a liquid sample between the first and second position electrodes, a signal can be sent to timer 308 to mark the time at which liquid is first present between the first and second position electrodes. When the measured impedance indicates that the liquid sample has reached the second position electrode, another signal can be sent to timer 308. The difference in time between when a liquid sample is first present between the first and second position electrodes and when the liquid sample reaches the second position electrode can be used to determine liquid sample flow rate (given knowledge of the volume of micro-channel 310 between the first and second position electrodes). Furthermore, knowledge of liquid sample flow rate and/or liquid sample position can be used to determine total liquid sample volume. In addition, a signal denoting the point in time at which a liquid sample arrives at second position electrode 304 can also be sent to a local controller module (e.g., local controller module 214 of FIG. 5) for use in determining the proper deformed state for membrane valve 130 and/or determining the retracted or deployed state of a sampling module's pressure ring. Therefore, such a local controller module can optionally include a feedback controller as described herein.

It can be desirable to collect a pre-determined minimum volume of ISF liquid sample before making an analytical measurement. Such a predetermined minimum volume may be in the range of, for example, from about 25 nanoliters to about 500 nanoliters, and preferably may range from about 100 nanoliters to about 250 nanoliters. In this regard, a sampling module pressure ring (such as pressure ring 228 of FIG. 5) can be placed in a deployed state for a duration of time such that the pre-determined volume of liquid is collected. Once first position electrode 302 and second position electrode 304 indicate that the pre-determined volume of liquid has been collected, a feedback controller within local controller module 214 can be employed to place the pressure ring(s) in a retracted state that essentially stops flow through the sampling module. It should be noted that under certain biological conditions, fluid may still flow through a sampling module even though pressure ring(s) 228 is in a retracted state. However, such flow is relatively insignificant in magnitude and typically ranges from, for example, 0 nanoliters per minute to 50 nanoliters per minute. If desired, the feedback controller can be employed to subsequently place the pressure ring(s) in a deployed state and, thus, restart liquid sample flow.

In an alternative embodiment to this invention, first position electrode 302 and second position electrode 304 may be used to measure flow rate instead of volume. In this case, local controller module 214, can employ a feedback controller that deploys or retracts pressure ring(s) based on flow rate.

FIG. 8A is a simplified cross-sectional and schematic diagram illustrating a manner in which a position electrode can be in operative communication with a microchannel in embodiments of microfluidic analytical systems according to the present invention. FIG. 8A depicts a micro-channel 350 (in cross-section), a micro-channel disc 352, a position electrode 354, a laminate layer 356 and a meter 358. In the configuration of FIG. 8A, position electrode 354 is in operative communication with the micro-channel 350 such that a surface 360 of position electrode 354 is exposed to liquid sample (depicted by the wavy lines in FIG. 8A) in micro-channel 350.

In the embodiment of FIG. 8A (and other embodiments of the present invention), micro-channel disc 352 and laminate layer 356 are made of electrically insulating material such as, for example, polymeric insulating materials (e.g., polystyrene, silicone rubber, PMMA, polycarbonate or PEEK) and non-polymeric insulating materials such as, for, example, glass.

FIG. 8B is a simplified cross-sectional and schematic diagram (employing the same labeling numerals as FIG. 8A) illustrating another manner by which a position electrode can be in operative communication with a micro-channel in embodiments of microfluidic analytical systems according to the present invention. FIG. 8B depicts a micro-channel 350 (in cross-section), a micro-channel disc 352, a position electrode 354, a laminate layer 356 and a meter 358. In the configuration of FIG. 8b, position electrode 354 is in operative communication with the micro-channel 350 but separated from micro-channel 350 by an insulating layer, namely a portion of laminate layer 356. A benefit of the manner depicted in FIG. 8B is that there is no direct contact between liquid sample in micro-channel 350 and position electrode 354 and, consequently, no electrolysis or electrochemical decomposition of the liquid sample due to position electrode 354 can occur.

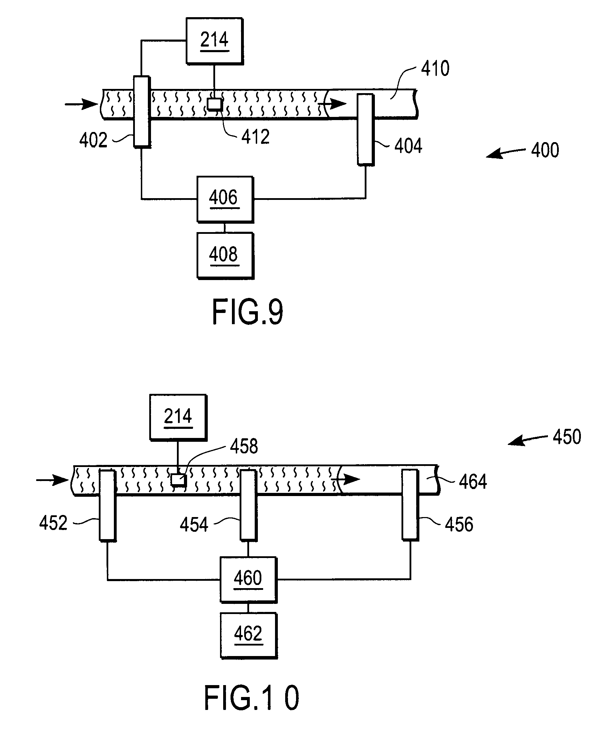

FIG. 9 is simplified schematic diagram of another micro-channel, analyte sensor and position electrode configuration 400 for use in embodiments of microfluidic analytical systems according to the present invention. Configuration 400 includes first position electrode 402, second position electrode 404, electrical impedance meter 406, timer 408, micro-channel 410 and analyte sensor 412. In the configuration of FIG. 9, wavy lines depict a liquid sample (e.g., an ISF, blood, urine, plasma, serum, buffer or reagent liquid sample) within micro-channel 410.

In the embodiment of FIG. 9, both first position electrode 402 and analyte sensor 412 are in operative communication with local controller module 214. In this manner first position electrode can serve both as a position electrode and as a reference electrode for analyte sensor 412 (assuming that analyte sensor 412 is an electrochemical-based analyte sensor). Furthermore, it should be noted that electrical impedance meter 406 and timer 408 may be incorporated into local controller module 214.

An advantage of the configuration of FIG. 9 is a reduced complexity that is achieved by using the first position electrode as both a position electrode and a reference electrode for analyte sensor 412. In the configuration of FIG. 9, first position electrode 402 can, for example, be manufactured of a material that results in a stable electrical potential between the first position electrode and the liquid sample. In the circumstance that the liquid sample is an ISF liquid sample, the first position electrode can be formed of chlorinated silver (Ag/AgCl).

FIG. 10 is a simplified schematic diagram of yet another position electrode, micro-channel, analyte sensor and meter configuration 450 for use in embodiments of microfluidic analytical systems according to the present invention. Configuration 450 includes first, second and third position electrodes 452, 454 and 456, respectively, an analyte sensor 458, an electrical impedance meter 460, timer 462, and micro-channel 464. Electrical impedance meter 460 is configured to measure the electrical impedance between any two of the first, second and third position electrodes.

Configuration 450 differs from configurations 300 and 400 in that configuration 450 includes three position electrodes. The inclusion of three position electrodes provides for an improved ability to accurately detect the position and flow rate of a liquid sample within micro-channel 464. For example, the use of two position electrodes enables the detection of a single bolus (i.e., the volume contained in a microchannel between the two position electrodes). However, the use of three (or more) position electrodes enables the detection of multiple boluses as the liquid sample sequentially passes the three (or more) position electrodes.

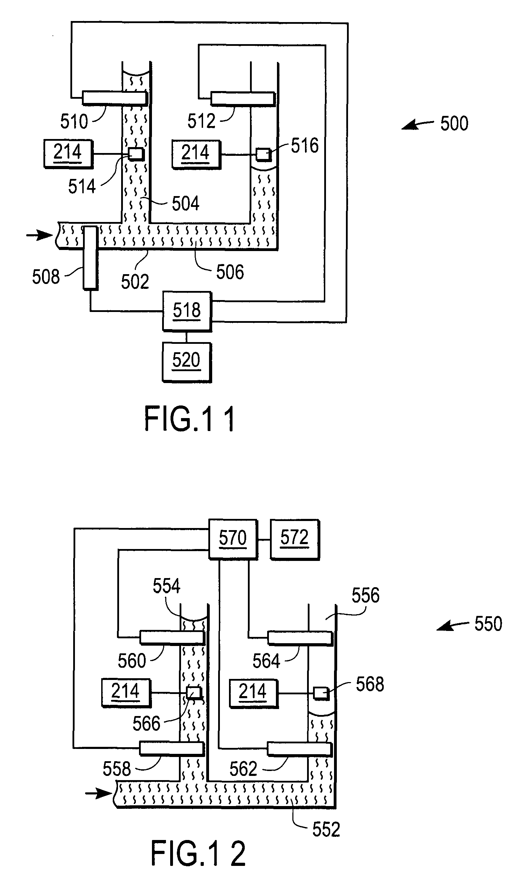

FIG. 11 is simplified schematic diagram of a position electrode, micro-channel (comprised of a main micro-channel and two branch micro-channels), analyte sensor and meter configuration 500 for use in embodiments of microfluidic analytical systems according to the present invention. Configuration 500 includes a microchannel comprised of main micro-channel 502, first branch micro-channel 504 and second branch micro-channel 506. Configuration 500 also includes first position electrode 508 (in operative communication with main micro-channel 502), second position electrode 510 (in operative communication with first branch micro-channel 504) and third position electrode 512 (in operative communication with second branch micro-channel 506).

Furthermore, configuration 500 includes a first analyte sensor 514 (in operative communication with first branch micro-channel 504) and a second analyte sensor 516 (in operative communication with second branch micro-channel 506), a meter 518 and timer 520. Meter 518 is configured to measure an electrical characteristic (e.g., impedance) between the first position electrode and either of the second and third position electrodes.

It is envisioned that configuration 500 will be employed in a device that includes liquid handling means for selectively directing a liquid sample from main micro-channel 502 to either of first and second branch micro-channels 504 and 506. Examples of such liquid handling means include, but are not limited to, active valves, passive valves, capillary breaks, air pressure barriers and hydrophobic patches.

Configuration 500 can be employed to detect the position of a liquid sample in either first branch micro-channel 504 (by employing meter 518 to measure an electrical characteristic between first position electrode 508 and second position electrode 510) or second branch micro-channel 506 (by employing meter 518 to measure an electrical characteristic between first position electrode 508 and third position electrode 512). Such detection(s) can be employed to control liquid sample flow and the determination of an analyte in the liquid sample by either first analyte sensor 514 or second analyte sensor 516.

FIG. 11 is simplified schematic diagram of another position electrode, microchannel (comprised of a main micro-channel and two branch micro-channels), analyte sensor and meter configuration 550 for use in embodiments of microfluidic analytical systems according to the present invention. Configuration 550 includes a microchannel comprised of main micro-channel 552, first branch micro-channel 554 and second branch micro-channel 556. Configuration 550 also includes first and second position electrodes 558 and 560 (in operative communication with first branch microchannel 554), and third and fourth position electrodes 562 and 564 (in operative communication with second branch micro-channel 556).

Furthermore, configuration 550 includes a first analyte sensor 566 (in operative communication with first branch micro-channel 554) and a second analyte sensor 568 (in operative communication with second branch micro-channel 556), a meter 570 and timer 572. Meter 570 is configured to measure an electrical characteristic (e.g., impedance) between either of the first and second position electrodes and the third and fourth position electrodes.

It is envisioned that configuration 550 will be employed in a device that includes liquid handling means for selectively directing a liquid sample from main micro-channel 552 to either of first and second branch micro-channels 554 and 556. Examples of such liquid handling means include, but are not limited to, active valves, passive valves, capillary breaks, air pressure barriers and hydrophobic patches.

Configuration 550 can be employed to detect the position of a liquid sample in either first branch micro-channel 554 (by employing meter 570 to measure an electrical characteristic between first position electrode 558 and second position electrode 560) or second branch micro-channel 556 (by employing meter 570 to measure an electrical characteristic between third position electrode 562 and fourth position electrode 564). Such detection(s) can be employed to control liquid sample flow and the determination of an analyte in the liquid sample by either first analyte sensor 566 or second analyte sensor 568. A benefit of configuration 550 is that the first and second position electrodes (as well as the third and fourth position electrodes) can be positioned relatively close together to enable accurate measurements of relatively high electrical characteristics (e.g., relatively high impedances) therebetween.

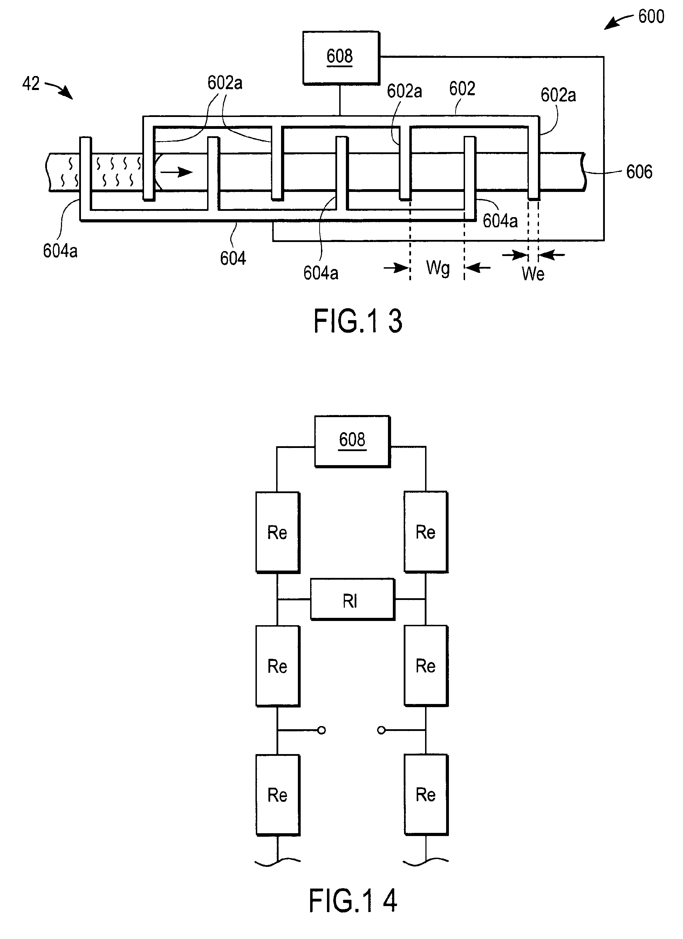

FIG. 13 is a simplified schematic diagram of a position electrode, microchannel and meter configuration 600 for use in embodiments of microfluidic analytical systems according to the present invention. FIG. 14 is a simplified schematic diagram of an equivalent electrical circuit for a portion of configuration 600 of FIG. 13.

Configuration 600 includes a first position electrode 602 and a second position electrode 604 in an interdigitated configuration. Configuration 600 also includes a micro-channel 606 and a meter 608. First and second position electrodes 602 and 604 each having a plurality of electrode portions that are placed substantially parallel to, and in alternating succession with, each other (e.g., in an alternating, "finger-like" pattern as depicted in FIG. 13). For explanatory purposes, four electrode portions for first and second position electrodes 602 and 604 (602a and 604a, respectively) are illustrated in FIG. 13. The interdigitated electrode portions are also referred to as "fingers."

The position electrodes of embodiments of the present invention and the spacing therebetween can be of any suitable dimension. Advantageously, an interdigitated configuration can be employed with dimensions (e.g., dimensions $W_g$ and $W_e$ of FIG. 13) that allow for the measurement of electrical properties of a relatively small liquid sample.

In configuration 600, each "finger" can independently have a width $W_e$ in the range of, for example, from about 1 micrometers to about 1500 micrometers. The separation between electrode "fingers" ($W_g$) can be, for example, in the range between about 0.1 millimeters and about 15 millimeters. The thickness of the position electrodes is sufficient to support a desired electric current. Exemplary thicknesses are, for example, in the range from about 1 micrometers to about 100 micrometers.

Interdigitated configurations such as configuration 600 can have any number of "fingers" that are sufficient to provide utility, e.g., providing contact with a liquid sample and to measure an electrical characteristic. An interdigitated configuration can have, for example, from 2 to about 100 "fingers."

Configuration 600 can be employed to detect a liquid sample bolus(es) flowing through micro-channel 606. With such boluses having a pre-determined volume (such as for example 250 nanoliters) defined by the height and width of micro-channel 606 and the distance $W_g$. For example, if micro-channel 606 has a height and width that are both about 250 microns, $W_e$ is about 0.5 millimeters and $W_g$ is about 4 millimeters, then when there is no liquid sample bridging between any finger of position electrode 602 and position electrode 604, the resistance between first electrode 602 and second electrode 604 is essentially infinity. However, if an ISF liquid sample bridges (fills) micro-channel 606 between the first finger of the first position electrode and the first finger of the second position electrode (a circumstance depicted by wavy lines in FIG. 13), a measured total resistance $R_T$ decreases to a liquid resistance $R_l$ of about 37 KOhm.

It should be noted that in configuration 600, the resistance of each finger $R_e$ is much less than $R_l$ by at least about a factor of ten. As micro-channel 606 fills further with a liquid sample, the measured total resistance $R_T$ between first position electrode 602 and second position electrode 604 further decreases. The decrease in total measured total resistance $R_T$ can characterized by the equation $R_T = \dfrac{R_l}{n}$, where $n$ = the number fingers "bridged" by the liquid sample. Configuration 600 is particularly useful when $R_e$ is much less than $R_l$.

In configuration 600, micro-channel 606 is depicted as passing (i.e., coming into operative communication with) each electrode finger 602a one time. However, micro-channel 606 could alternatively have a serpentine configuration such that microchannel 606 passes each electrode finger 602a a plurality of times. Such a configuration can enhance the ability to easily resolve relatively small liquid sample volumes (e.g., liquid sample volumes of less than 5 nl).

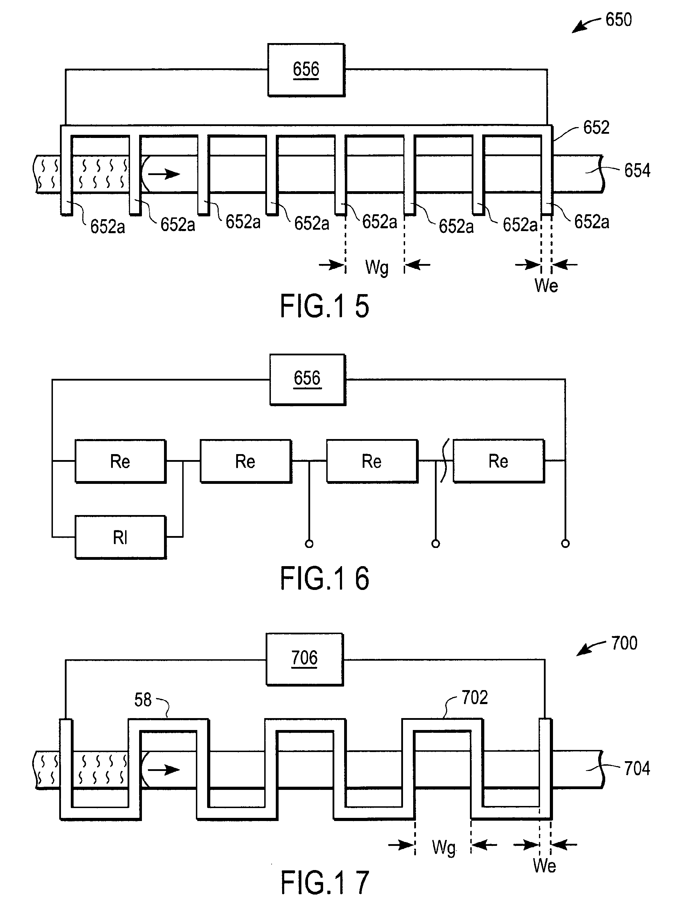

FIG. 15 is a simplified schematic diagram of a position electrode, microchannel and meter configuration 650 for use in embodiments of microfluidic analytical systems according to the present invention. FIG. 16 is a simplified schematic diagram of an equivalent electrical circuit for a portion of configuration 600 of FIG. 15.

Configuration 650 includes a single comb-shaped position electrode 652 with eight "fingers" 652a, a micro-channel 654 and a meter 656. Electrode fingers 652a serve to define electrode segments therebetween with each segment having a resistance $R_e$ (as depicted in FIG. 16). It should be noted that the dimensions $W_g$ and $W_e$ of FIG. 16 can be the same as described previously with respect to configuration 600.

When there is no liquid sample in micro-channel 654 between any of the eight fingers 652a, a measured total resistance of position electrode 652 is the summation of the resistance for each electrode segment $R_e$ (i.e., the resistance of all electrode elements together). However, once a liquid sample begins to fill micro-channel 654 between any of fingers 652a, the measured total resistance $R_T$ decreases since resistance $R_l$ is created in parallel to $R_e$ (see FIG. 16). It should be noted that with respect to configuration 650, the resistance of each electrode segment $R_e$ is significantly greater than $R_l$, preferably by about a factor of ten or greater.

FIG. 17 is a simplified schematic diagram of a position electrode, microchannel and meter configuration 700 for use in embodiments of microfluidic analytical systems according to the present invention. Configuration 700 includes a single serpentine-shaped position electrode 702, a micro-channel 704 and a meter 706.

It should be noted that the dimensions $W_g$ and $W_e$ of FIG. 16 can be the same as described previously with respect to configuration 600.

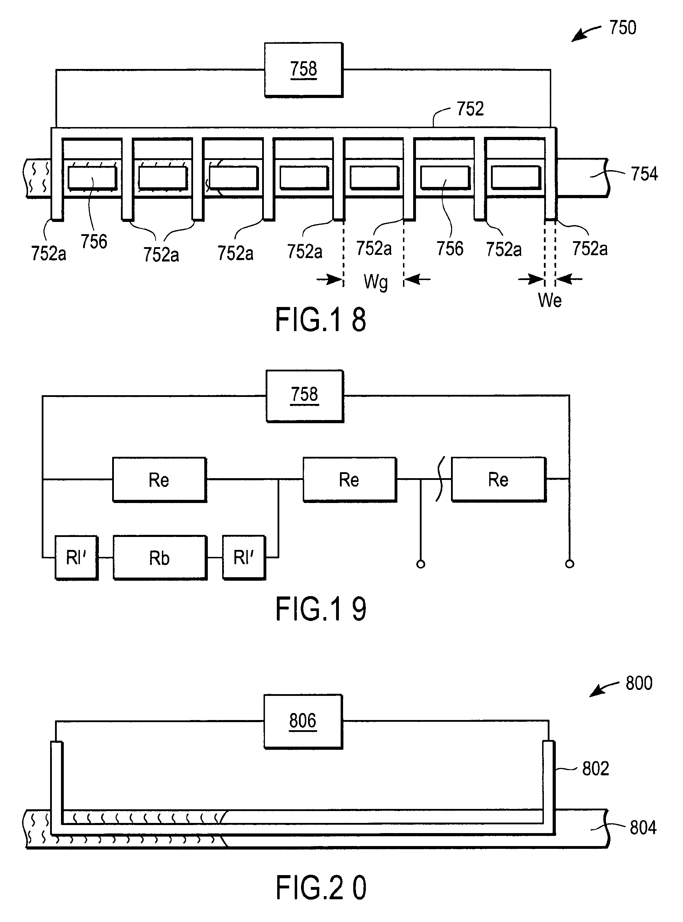

FIG. 18 is a simplified schematic diagram of a position electrode, microchannel and meter configuration 750 for use in embodiments of microfluidic analytical systems according to the present invention. FIG. 19 is a simplified schematic diagram of an equivalent electrical circuit for a portion of configuration 750 of FIG. 18.

Configuration 750 includes a position electrode 752, micro-channel 754, bypass electrode 756, and meter 758. Position electrode 752 is a single comb-shaped position electrode with eight electrode "fingers" 752a. Electrode fingers 752a serve to define electrode segments therebetween with each segment having a resistance $R_e$ (as depicted in FIG. 18). It should be noted that the dimensions $W_g$ and $W_e$ of FIG. 18 can be the same as described previously with respect to configuration 600.

In the absence of any liquid sample, bypass electrode 756 is electrically floating. However, when a liquid sample is present between two consecutive electrode fingers 752a, bypass electrode 756 becomes a part of the circuit depicted in FIG. 19 and is characterized by resistance $R_b$.

Assuming that $R_b$ is significantly less than $R_l'$ (i.e., the resistance of a liquid sample between an electrode finger and a bypass electrode), more current will flow through the bypass electrode than the liquid sample. Therefore, configuration 750 is beneficial when used in combination with high-resistive liquid samples since bypass electrode 756 effectively reduces the $R_T$, as shown schematically in FIG. 19. Furthermore, once apprised of the present disclosure, one skilled in the art will recognize that a bypass electrode(s) can be similarly disposed between position electrodes or between electrode fingers in a variety of electrode configurations (for example, the configurations of FIGs. 7, 9-13 and 17) to reduce total measured resistance in the presence of a relatively high-resistive liquid sample.

FIG. 20 is a simplified schematic diagram of a position electrode, microchannel and meter configuration 800 for use in embodiments of microfluidic analytical systems according to the present invention. Configuration 800 includes a position electrode 802, micro-channel 804 and meter 806. Meter 806 is configured to measure a continually changing electrical characteristic of position electrode 802 as a liquid sample (depicted by the wavy lines in FIG. 20) passes through micro-channel 804.

EXAMPLE

An interdigitated configuration similar to that of FIG. 13 was tested by employing a phosphate buffer solution as a liquid sample. The first and second position electrodes of the configuration were formed from Ag/AgCl using a screen printing technique. In addition, the first position electrode and second position electrode were separated by a distance $W_g$ of 4 millimeters.

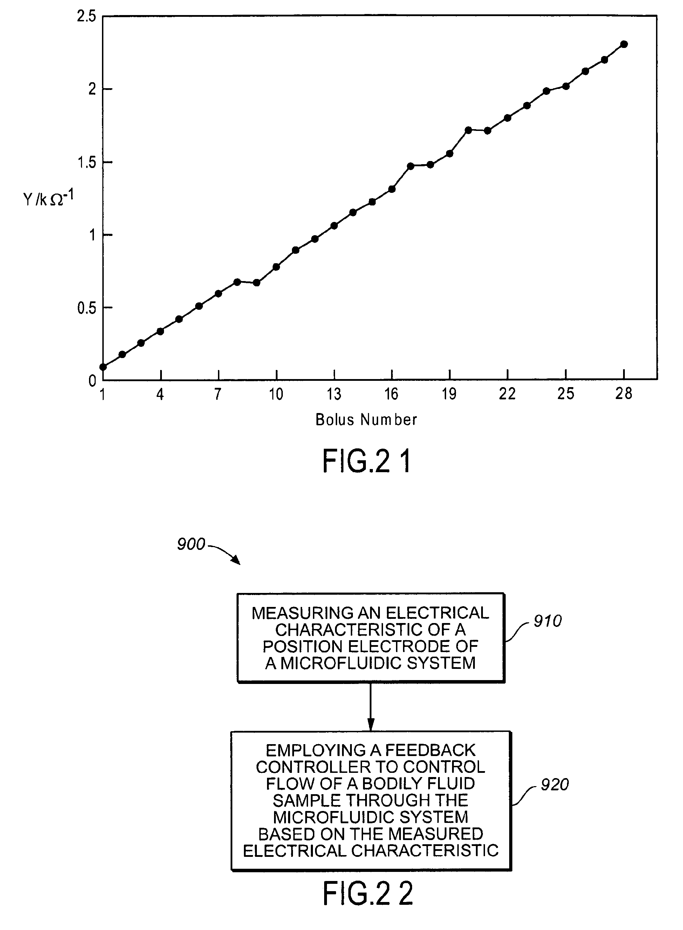

A potential waveform was applied between the first and second position electrodes with a frequency of 0.25 MHz, an amplitude of +/-0,1 volts, and a RMS of 0 volt. Based on the resulting current between the first and second position electrodes, measured total resistance $R_T$ and total measured admittance were calculated (it should be noted that AT = $1/R_T$). FIG. 21 shows that the measured total admittance AT increases linearly as successive liquid sample boluses pass each of the electrode fingers of the configuration.

FIG. 21 illustrates that each successive bolus was detected as a change in admittance. Therefore, boluses can be counted by, for example, monitoring for spikes in the derivative of a measured impedance signal versus time.

FIG. 22 is a flow diagram depicting stages in a method 900 for the feedback control of a microfluidic system. Method 900 includes measuring an electrical characteristic (such as impedance or resistance) of at least one position electrode of a microfluidic system with a meter of the microfluidic system, as set forth in step 910. One skilled in the art will recognize that such a microfluidic system can be any suitable microfluidic system embodiment described herein including, but not limited to, the embodiments described with respect to FIG. 5 and related figures.

At step 920, the feedback controller is employed to control the flow of bodily fluid sample through the microfluidic system based on the electrical characteristic measured by the meter. As discussed above with respect to embodiments of microfluidic systems and microfluidic analytical systems according to the present invention, such control can be accomplished by (i) placing a pressure ring in a retracted state or a deployed state or (ii) by placing a membrane valve in a deformed or undeformed state. In addition, the control can be based on electrical characteristics that are indicative of a liquid sample's position, flow rate and/or volume. Furthermore, the flow can be controlled to facilitate semi-continuous stopped flow measurements.

It should be understood that various alternatives to the embodiments of the invention described herein may be employed in practicing the invention. It is intended that the following claims define the scope of the invention and that methods and structures within the scope of these claims and their equivalents be covered thereby.

**Claims**

1. A method for the feedback control of a microfluidic system, the method comprising:

   measuring an electrical characteristic of at least one position electrode of a microfluidic system with a meter of the microfluidic system, the microfluidic system including:

   an analysis module with at least one micro-channel for receiving and transporting a bodily fluid sample; at least one analyte sensor for measuring an analyte in the bodily fluid sample, each of the at least one analyte sensors being in operative communication with a micro-channel; and

the at least one position electrode, each of the at least one position electrodes in operative communication with at least one micro-channel;

a sampling module configured to extract the bodily fluid sample from a target site of a user's body and transport the bodily fluid sample to the at least one microchannel;

a meter configured for measuring an electrical characteristic of the at least one position electrode, the electrical characteristic being dependent on a position of the liquid sample in the micro-channel in operative communication with the at least one position electrode for which an electrical characteristic is measured; and

a feedback controller adapted to control the flow of bodily fluid sample through the microfluidic system based on the electrical characteristic measured by the meter; and

employing the feedback controller to control the flow of bodily fluid sample through the microfluidic system based on the electrical characteristic measured by the meter.

2. The method of claim 1, wherein the measuring step measures an impedance of the at least one position electrode.

3. The method of claim 1, wherein the measuring step measures a resistance of the at least one position electrode.

4. The method of claim 1, wherein the employing step employs the feedback controller to control the flow of bodily fluid sample through the microfluidic system based on the electrical characteristic measured by the meter that is indicative of a position of the bodily fluid sample.

5. The method of claim 1, wherein the employing step employs the feedback controller to control the flow of bodily fluid sample through the microfluidic system based on the electrical characteristic measured by the meter that is indicative of a flow rate of the bodily fluid sample.

6. The method of claim 1, wherein the employing step employs the feedback controller to control the flow of bodily fluid sample through the microfluidic system based on the electrical characteristic measured by the meter that is indicative of a volume of the bodily fluid sample.

7. The method of claim 1, wherein the employing step employs the feedback controller to control the flow of bodily fluid sample through the microfluidic system by placing a pressure ring of the sampling module in at least one of a retracted state and a deployed state.

8. The method of claim 1, wherein the employing step employs the feedback controller to control the flow of bodily fluid sample through the microfluidic system by placing a membrane valve of the analysis module in at least one of a deformed condition and an undeformed condition.

9. The method of claim 1, wherein the bodily fluid sample is an interstitial fluid (ISF) sample.

10. The method of claim 1, wherein the measuring step measures the electrical characteristic employing an electrical signal with a frequency in the range of from about 10 Hz to about 100 kHz and a voltage waveform symmetrical around 0 Volts.

11. The method of claim 1, wherein the feedback controller controls the flow of bodily fluid sample through the microfluidic system based to facilitate semi-continuous stopped flow measurements of an analyte in the bodily fluid sample by the analyte sensor.

FIG.1

FIG.2

114

120 122

FIG.3

116

124 104B 105 104A

126

FIG.4

118

110 108 106 128

130

**FIG.5**

**FIG.6**

FIG.7

FIG.8 A

FIG.8 B

214

410

402 412 404 400

406

408

FIG.9

214

450

458

452 454 456 464

460

462

FIG.10

FIG.1 1

FIG.1 2

FIG.1 3

FIG.1 4

650

656

652

654

652a 652a 652a 652a 652a 652a 652a 652a

Wg

We

## FIG.1 5

656

Re Re Re Re

Rl

## FIG.1 6

700

706

58 702

704

Wg

We

## FIG.1 7

FIG.1 8

FIG.1 9

FIG.2 0

FIG.2 1

FIG.2 2

**European Patent Office**

**EUROPEAN SEARCH REPORT**

Application Number

EP 06 25 3938

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/50534 A1 (INVERNESS MEDICAL LTD [GB]; RICHTER TANJA [GB]; ALLEN JOHN [US]; MCALE) 27 June 2002 (2002-06-27) * page 8, line 30 - page 9, line 5 * * page 10, line 1 - line 12 * * page 15, line 37 - page 16, line 8 * | 1-4,9,10 | INV. B01L3/00 G01N33/487 A61B5/00 |
| A | DE 101 22 133 A1 (AGILENT TECHNOLOGIES INC [US]) 10 January 2002 (2002-01-10) * paragraph [0028] - paragraph [0029] * * paragraph [0038] - paragraph [0041] * | 1-11 | |
| A | SCHOLER L ET AL: "Monolithically integrated micro flow sensor for lab-on-chip applications" MICROELECTRONIC ENGINEERING, ELSEVIER PUBLISHERS BV., AMSTERDAM, NL, vol. 78-79, March 2005 (2005-03), pages 164-170, XP004803615 ISSN: 0167-9317 * abstract * | 1-11 | |
| A | BOHM S ET AL: "A plastic micropump constructed with conventional techniques and materials" SENSORS AND ACTUATORS A, ELSEVIER SEQUOIA S.A., LAUSANNE, CH, vol. 77, no. 3, 2 November 1999 (1999-11-02), pages 223-228, XP004244569 ISSN: 0924-4247 * abstract * | 1-11 | TECHNICAL FIELDS SEARCHED (IPC) B01L G01N A61B |

The present search report has been drawn up for all claims

| Place of search | Date of completion of the search | Examiner |
|---|---|---|
| Munich | 25 October 2006 | Komenda, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 749 575 A1

**ANNEX TO THE EUROPEAN SEARCH REPORT
ON EUROPEAN PATENT APPLICATION NO.**

EP 06 25 3938

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2006

| Patent document cited in search report | Publication date | Patent family member(s) | Publication date |
|---|---|---|---|
| WO 0250534 A1 | 27-06-2002 | AU 2227502 A | 01-07-2002 |
| | | AU 2228002 A | 01-07-2002 |
| | | CA 2432452 A1 | 27-06-2002 |
| | | CA 2432535 A1 | 27-06-2002 |
| | | CN 1491358 A | 21-04-2004 |
| | | CN 1499949 A | 26-05-2004 |
| | | EP 1359837 A1 | 12-11-2003 |
| | | EP 1350099 A1 | 08-10-2003 |
| | | WO 0249507 A1 | 27-06-2002 |
| | | JP 2004520103 T | 08-07-2004 |
| | | JP 2004522146 T | 22-07-2004 |
| | | US 2004072357 A1 | 15-04-2004 |
| | | US 2004096959 A1 | 20-05-2004 |
| DE 10122133 A1 | 10-01-2002 | US 6632400 B1 | 14-10-2003 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 10811446 A
- GB 0105634 W
- WO 0249507 A1
- US 653023 A
- US 185605 A
- US 20030060784 A1
- US 718818 A